(19) Europäisches Patentamt
European Patent Office
Office européen des brevets

(11) Numéro de publication: **0 089 895**
**A2**

(12) # DEMANDE DE BREVET EUROPEEN

(21) Numéro de dépôt: 83400579.5

(22) Date de dépôt: 21.03.83

(51) Int. Cl.[3]: B 01 J 23/74
B 01 J 37/30, C 07 C 2/24
B 01 J 31/16

(30) Priorité: 23.03.82 FR 8204888

(43) Date de publication de la demande: 28.09.83 Bulletin 83/39

(84) Etats contractants désignés: DE FR GB IT

(71) Demandeur: Etablissement Public dit: CENTRE NATIONAL DE LA RECHERCHE SCIENTIFIQUE (CNRS)
15, Quai Anatole France
F-75007 Paris(FR)

(72) Inventeur: Bonneviot, Laurent
1 rue de la Collégiale
F-75005 Paris(FR)

(72) Inventeur: Olivier, Danielle
Domaine du Réveillon 6 rue du Gré
F-94440 Villecresnes(FR)

(72) Inventeur: Che, Michel
12 avenue F. Roosevelt
F-923300 Sceaux(FR)

(74) Mandataire: Ahner, Francis et al,
CABINET REGIMBEAU 26, avenue Kléber
F-75116 Paris(FR)

(54) **Nouveau catalyseur d'oligomérisation à base de nickel monovalent Ni+ sur support de silice, sa préparation et les procédés d'oligomérisation en présence de ce catalyseur.**

(57) La présente invention concerne un nouveau catalyseur d'oligomérisation à base de nickel monovalent $Ni^+$ sur support de silice, sa préparation et les procédés d'oligomérisation en présence de ce catalyseur.

Le catalyseur selon l'invention se caractérise en ce qu'il présente des précurseurs de sites d'activité catalytique, constitués par des complexes de nickel monovalent $Ni^+$ tricoordiné à trois atomes d'oxygène de la surface du support de silice.

EP 0 089 895 A2

NOUVEAU CATALYSEUR D'OLIGOMERISATION A BASE DE NICKEL MONOVALENT $Ni^+$ SUR SUPPORT DE SILICE, SA PREPARATION ET LES PROCEDES D'OLIGOMERISATION EN PRESENCE DE CE CATALYSEUR.

La présente invention concerne un nouveau catalyseur d'oligomérisation et plus particulièrement de dimérisation d'oléfines, à base de nickel monovalent $Ni^+$ sur support de silice, ainsi que sa préparation et les procédés d'oligomérisation mis en oeuvre en présence de ce catalyseur.

Dans la technique antérieure, on connaît déjà un certain nombre de catalyseurs hétérogènes à base de nickel et de silice, qui sont susceptibles de dimériser les oléfines. Comme décrit par SOHN et OSAKI dans Journal of Catalysis 59, 303,1979, ces catalyseurs sont le plus souvent obtenus par co-précipitation en milieu basique d'un silicate et d'un sel de nickel, suivie d'une activation par chauffage entre 100 et 600°C. Ces catalyseurs ne présentent cependant qu'une faible activité, car le nickel se trouve à l'intérieur de la silice sous une forme difficilement réductible.

Il a maintenant été trouvé qu'un catalyseur plus actif peut être obtenu par mise en oeuvre d'un mode de préparation permettant précisément d'éviter le passage par un composé de nickel difficilement réductible.

Le catalyseur selon la présente invention se trouve caractérisé par la présence de précurseurs de sites d'activité catalytique, constitués par des complexes de nickel monovalent $Ni^+$ tricoordonné à trois atomes d'oxygène de la surface du support de silice.

Selon une autre caractéristique de la présente invention le catalyseur contient d'environ 0,01 % à environ 1 % en poids sec de $Ni^+$.

Selon une forme de réalisation avantageuse les complexes de nickel monovalent $Ni^+$ peuvent en outre comprendre de 1 à 3 ligand(s) de blocage sélectif.

Conformément à la présente invention, le catalyseur d'oligomérisation est caractérisé par le fait qu'il est préparé par la mise en oeuvre des différentes étapes successives suivantes :

a) on traite la silice par une solution aqueuse d'un composé générateur de cations monovalents, à un pH supérieur à environ 10, de manière à fixer au moins une partie desdits cations monovalents sur la silice ;

b) on traite la silice ainsi obtenue par une solution aqueuse d'un sel de nickel divalent et d'un agent complexant du sel de nickel à un pH au moins égal à environ 8, de manière à échanger lesdits cations monovalents portés par la silice avec des cations de nickel divalents ;

c) on porte la silice ainsi modifiée à une température comprise entre environ 300 et environ 900°C, et

d) on traite la silice résultante par un agent réducteur de manière à convertir au moins une partie du nickel divalent en nickel monovalent.

La valence du nickel fixé sur la silice, conformément à la présente invention, a pu être déterminée par mesure de résonance paramagnétique électronique. Le nickel monovalent ($Ni^{+}$) se caractérise par un signal de type axial. Les valeurs mesurées ont été d'environ 2,76 pour un g parallèle et d'environ 2,097 pour un g perpendiculaire.

D'autres caractéristiques et avantages de la présente invention apparaîtront à la lecture de la description détaillée faite ci-après.

Le catalyseur d'oligomérisation selon l'invention est obtenu par la mise en oeuvre d'un procédé qui se décompose en quatre étapes successives, présentées ci-après de façon détaillée.

a) <u>Fixation de cations monovalents sur une silice</u>

On traite la silice par une solution aqueuse d'un composé fournissant, en solution aqueuse, des cations monovalents, notamment des cations de métaux alcalins, d'ammonium ou d'ammonium quaternaire, par exemple $NH_4^+$, $Na^+$, $Li^+$, $K^+$ ou $NR_4^+$ (R = reste monovalent hydrocarboné), à un pH supérieur à environ 10, de préférence entre environ 11 et environ 11,8.

Des exemples de composés fournissant des cations monovalents en solution, dans le domaine de pH précité, sont la soude, la potasse, l'ammoniaque et les hydroxydes d'ammonium quaternaire, avec ou sans sels additionnels, par exemple le chlorure d'ammonium, le nitrate de sodium,

le chlorure de lithium, l'acétate d'ammonium ou un chlorure ou sulfate d'ammonium quaternaire.

Cette étape de fixation est avantageusement effectuée à une température comprise entre environ 0 et environ 80°C, de préférence entre environ 20 et environ 60°C, en opérant, par exemple, dans un récipient sous agitation ou par circulation de la solution aqueuse au travers d'un lit de silice. La durée du traitement peut s'étendre, par exemple, de 3 heures à 3 jours.

Au cours de cette étape de fixation, le pH doit demeurer supérieur à environ 10 et de préférence être compris entre environ 11 et environ 11,8, ce qui peut nécessiter dans certains cas l'addition complémentaire d'une base telle que la soude, la potasse ou l'ammoniaque si l'on constate une baisse excessive du pH. Pour éviter cet inconvénient, une méthode préférée consiste à utiliser la solution aqueuse en quantité et concentration suffisantes pour que le pH ne baisse pas de plus d'environ 0,3 unité .

Pour ce premier traitement, la silice peut être mise en oeuvre sous une forme quelconque, et notamment sous forme de poudre, de billes ou d'extrudés.

b) Echange compétitif des cations monovalents avec le cation $Ni^{2+}$

La silice traitée comme indiqué ci-dessus et qui renferme des cations monovalents est traitée par une solution aqueuse d'un sel de nickel bivalent et d'un agent complexant du sel de nickel, à un pH d'au moins environ 8, et de préférence compris entre environ 9 et environ 11,8. L'agent complexant basique est avantageusement l'ammoniac ou une amine, par exemple la pyridine, la monoéthyl amine, la diéthyl amine ou la triméthyl amine.

La solution contient, par exemple d'environ 0,001 à environ 1 mole de sel de nickel par litre, la concentration et la quantité de solution étant choisies en fonction du nombre d'ions monovalents qu'on désire échanger de manière compétitive. Pour donner un ordre de grandeur, un litre de cette solution permet de traiter entre environ 1 et environ 100 g de silice. La durée du traitement est comprise par exemple entre environ 3 heures et environ 3 jours, selon le taux d'échange compétitif désiré, et peut se faire par exemple dans un récipient sous agitation ou par circulation de la solution au travers d'un lit de silice. La température de l'échange compétitif est avantageusement comprise entre environ 0 et environ 80°C. La silice modifiée ainsi obtenue est séparée de la phase aqueuse et lavée à l'eau. Elle contient par exemple, entre environ 0,01 et environ 10 % en poids sec de nickel.

Il est préférable de limiter le taux d'échange compétitif en nickel à environ 7 % afin d'éviter la formation de nickel métallique au cours de la réduction ultérieure.

c) Prétraitement thermique

La silice obtenue au cours de l'étape précédente est ensuite portée à une température comprise entre environ 300 et environ 900°C, de préférence entre environ 500 et environ 700°C. On peut opérer sous vide, dans un gaz inerte, tel que l'azote ou l'argon, ou de préférence dans un gaz oxydant, tel que l'oxygène pur ou un mélange d'oxygène avec un gaz inerte. La montée en température est de préférence progressive, sa vitesse étant, par exemple, comprise entre environ 10 et environ 500°C par heure. La durée du prétraitement thermique est, par exemple d'environ 1 à environ 12 heures. Ce traitement permet d'éliminer l'eau contenue dans la silice. Si le

chauffage de la silice se fait par l'intermédiaire de la phase gazeuse le débit de gaz est réglé en fonction de la température à atteindre. A la fin du prétraitement thermique, la silice est refroidie à l'abri de l'humidité.

Il y a en outre lieu d'observer que les durées relatives, et les températures des prétraitements thermiques sous vide et sous oxygène influent :

. sur la réductibilité des ions $Ni^{2+}$ en ions $Ni^{+}$, et

. sur la sélectivité ultérieure du catalyseur pour la dimérisation de l'éthylène.

Les conditions opérationnelles suivantes ont par exemple été retenues :

i) les échantillons sont chauffés sous courant d'oxygène sec à 100°C/heure jusqu'à 500°C et ne doivent pas être maintenus plus de 2 heures sous débit d'oxygène à cette température,

ii) un traitement sous vide, 20 heures à 500°C, conduit à un catalyseur sélectif pour la dimérisation de l'éthylène, et

iii) les catalyseurs évacués 20 heures à 700°C dimérisent et trimérisent l'éthylène en proportions sensiblement équivalentes.

Le tableau I ci-après illustre bien l'influence de la température de prétraitement thermique de l'étape c) sur la sélectivité ultérieure du catalyseur obtenu après l'étape de réduction d).

TABLEAU I

| Température de prétraitement sous vide °C | éthylène | 1-butène | 2-butène | hexène |
|---|---|---|---|---|
| | % molaires | | | |
| 500 | 9,9 | 5,8 | 77,2 | 7,1 |
| 700 | 8,6 | 2 | 43,2 | 46,2 |

Conditions expérimentales : T = 25°C ; $P_{C_2H_4} = 8.10^4$ Pa temps de réaction = 24 heures (le catalyseur a en outre subi une réduction photochimique, comme indiqué ci-après)

d) Réduction

La silice obtenue au terme du prétraitement thermique est ensuite soumise à une réduction, laquelle peut être conduite en phase gazeuse ou en phase liquide.

En phase gazeuse, le réducteur peut être par exemple, l'hydrogène, le monoxyde de carbone, l'oxyde azoteux ou l'éthylène, purs ou en mélange avec un gaz inerte comme l'azote ou l'argon. La réduction peut se faire en système fermé ou par passage d'un courant de gaz au travers d'un lit de silice, sous une pression de, par exemple environ 10 à environ $10^6$ Pascals. La température est comprise, de préférence entre environ 100 et environ 300°C, une température plus élevée conduisant à la formation de nickel métallique. La réduction peut durer, par exemple d'environ 1 à environ 24 heures. L'irradiation UV par une lampe à vapeur de mercure haute pression permet de conduire la réduction à basse température (d'environ -196°C à environ 27°C) et d'éviter ainsi toute formation de nickel métallique.

Après environ 30 heures de réduction plus de la moitié des ions $Ni^{2+}$ sont réduits à l'état d'ions $Ni^+$.

En phase liquide, la réduction peut se faire par un composé organique comme l'hydrazine ou par un métal alcalin, par exemple le lithium, le sodium ou le potassium, ou par un hydrure ou un dérivé organométallique de l'un des métaux précités, en solution dans l'ammoniac liquide ou dans tout autre milieu polaire aprotique, par exemple dans un éther.

Le catalyseur ainsi obtenu renferme du nickel à l'état de $Ni^+$ (contrôle par mesure de résonance paramagnétique électronique). Conservé à l'abri de l'humidité, il se montre très actif pour l'oligomérisation des oléfines, utilisées seules ou en mélange, par exemple l'éthylène, le propylène et/ou les n-butènes.

Selon une forme de réalisation avantageuse du catalyseur d'oligomérisation selon l'invention, les complexes de $Ni^+$ comprennent en outre de 1 à 3 et de préférence 1 ligand(s) de blocage sélectif. Ces ligands qui sont par exemple avantageusement choisis parmi les phosphines telles que les tri-alcoyl phosphines et les tri-aryl phosphines, en particulier la triéthyl phosphine et la triphényl phosphine, bloquent chacun une place libre de la sphère de coordination du $Ni^+$. Il en résulte, lors de l'utilisation ultérieure du catalyseur, une activation et une meilleure sélectivité de la réaction de dimérisation.

La fixation des ligands de blocage sélectif est réalisée par adsorption sous une pression d'environ 500 Pa à environ 2000 Pa. Cette fixation de ligands sera habituellement réalisée postérieurement à l'étape de réduction d). Il en sera ainsi en particulier dans le cas d'une fixation de phosphines. Il est cependant

également possible de conduire simultanément les deux étapes de réduction et de fixation des ligands de blocage sélectif. Dans pareil cas, il est bien sûr nécessaire de choisir un ligand doté de propriétés réductrices, tel que par exemple l'oxyde azotique NO qui permet d'effectuer à 25°C la réduction du nickel divalent selon la réaction d'équilibre :

$$Ni^{2+} + NO \rightleftharpoons Ni^{+}NO^{+}$$

L'invention se rapporte également à un procédé d'oligomérisation faisant intervenir un catalyseur tel que défini précédemment. L'oligomérisation s'effectue par mise en contact de l'oléfine avec le catalyseur. L'oléfine peut être utilisée en phase gazeuse ou en phase liquide, à l'état pur ou en solution dans un solvant. Les meilleurs solvants de la réaction sont les hydrocarbures paraffiniques ou aromatiques, ou leurs dérivés chlorés, tels le pentane, l'heptane, le benzène, le toluène, le chlorure de méthylène, le dichloréthane, le chlorobenzène.

La température de dimérisation est avantageusement comprise entre environ 0° et environ 120°C, de préférence entre environ 20 et environ 60°C et la pression est choisie pour maintenir une phase gazeuse ou liquide. La dimérisation peut être conduite en système fermé (on introduit en une seule fois le catalyseur et l'oléfine dans le réacteur), en système semi-ouvert (on introduit progressivement l'oléfine dans un réacteur contenant le catalyseur, au fur et à mesure de sa réaction), ou en système ouvert (on fait passer l'oléfine au travers d'un lit du catalyseur). Le temps de contact entre le réactif et le catalyseur est usuellement compris entre environ 1 minute et environ 24 heures.

Quand, après un certain usage, le catalyseur voit son activité décroître, il peut être régénéré en reprenant le traitement thermique et la réduction tels que décrits précédemment.

Le catalyseur selon l'invention peut également être utilisé pour l'oligomérisation des alcynes.

Les exemples suivants illustrent l'invention sans cependant en limiter la portée.

A/ Préparation du catalyseur

Exemple 1

30 g de silice sont traités à température ambiante par 1 litre de solution aqueuse d'ammoniac à pH 11,6, sous faible agitation pendant 24 heures. On vérifie que la baisse du pH est restée inférieure à 0,3 unité . La silice utilisée est : soit une silice microporeuse (diamètre moyen des pores 5 nm) soit une sphérosil (diamètre moyen des pores 8 nm), les deux silices étant commercialisées par la Société Rhône-Poulenc. Après filtration les échantillons ainsi traités sont ensuite mis en contact à température ambiante avec 1 litre d'une solution contenant des ions $Ni(NH_3)_6^{2+}$, obtenue par dissolution de 0,05 mole de nitrate de nickel dans une solution aqueuse contenant 0,3 mole de nitrate d'ammonium et 1,5 mole d'ammoniac et dont le pH est voisin de 9,8. L'agitation est poursuivie pendant 60 heures à température ambiante. La silice est filtrée puis lavée à l'eau déionisée, séchée à 50°C sous vide. L'analyse élémentaire montre que sa teneur en nickel est de 1,7 % en poids sec.

Exemple 2

17 g de silice sont traités par une solution aqueuse d'ammoniac dans les conditions décrites dans la première étape de l'exemple 1. Après filtration, la silice est

mise dans 1 litre d'une solution contenant des ions $Ni(NH_3)_6^{2+}$ préparée à partir de la dissolution de 0,1 mole de nitrate de nickel et de 0,6 mole de nitrate d'ammonium dans une solution aqueuse contenant 4 moles d'ammoniac. La solution a un pH voisin de 10,4. Après 60 heures d'agitation à température ambiante la silice est lavée, filtrée et séchée comme dans l'exemple 1. Elle titre 4,2 % en nickel pour le support de silice microporeuse RP sous forme d'extrudés et 4,9 % en nickel dans le cas du support spherosil Pechiney-St-Gobain.

B/ Réduction du catalyseur avant réaction

Les échantillons 1 et 2 sont placés dans un tube sous un courant d'oxygène sec et chauffés progressivement jusqu'à 500°C pour l'échantillon 1 et 700°C pour l'échantillon 2. Le tube est ensuite mis sous vide et refroidi à température ambiante.

Exemple 1

L'échantillon 1 (1,7 % de Ni) est réduit soit thermiquement, soit photochimiquement.

La réduction thermique est effectuée sous un courant d'hydrogène sec à une pression de 50 kPa.

La température est fixée entre 170° et 220°C.

Après 20 heures de passage d'hydrogène, le tube est à nouveau mis sous vide pour chasser l'hydrogène.

Le catalyseur est ensuite conservé sous vide ou sous atmosphère d'argon.

La réduction photochimique est effectuée, dans un tube de quartz fermé, par de l'hydrogène sec à la pression de 50 kPa. Le tube de quartz est immergé dans l'azote liquide et irradié 3 heures par le rayonnement d'une lampe à vapeur de mercure haute pression. Après

réduction l'hydrogène est évacué à température ambiante. Le catalyseur est conservé sous vide ou sous argon.

Un examen par résonance paramagnétique électronique montre que les deux modes de réduction conduisent à un taux de réduction du $Ni^{2+}$ en $Ni^{+}$ qui peut atteindre jusqu'à environ 10%.

Exemple 2 (teneur en $Ni^{2+}$ 4,2 et 4,9 %)

L'échantillon est réduit photochimiquement suivant le processus décrit dans l'exemple 1. La cinétique de réduction est suivie par RPE. Après 15 heures de réduction environ 7 % des ions $Ni^{2+}$ sont réduits à l'état d'ions $Ni^{+}$.

C/ Dimérisation de l'éthylène

La dimérisation de l'éthylène a été étudiée sur la silice décrite dans l'exemple 1 (teneur en $Ni^{2+}$ 1,7 %). Les résultats sont identiques pour les échantillons réduits thermiquement ou photochimiquement.

1) Dimérisation en système fermé (régime statique)

20 mg de catalyseur réduit sont placés dans un tube de verre, en opérant à l'abri de l'air et de l'humidité.

Après avoir fait le vide on introduit l'éthylène sous une pression de 100 kPa. Après 24 heures de réaction à 25°C, la conversion de l'éthylène atteignait 98 % avec une sélectivité en butènes de 95,5 %. Le rapport $\frac{\text{butène -2}}{\text{butène -1}}$ était voisin de 48 avec un rapport

$\frac{\text{butène 2-trans}}{\text{butène 2-cis}}$ égal à 3.

2) Dimérisation en système ouvert (régime dynamique)

Sur 80 mg de catalyseur, on fait passer à 32°C un mélange d'éthylène et d'argon : le débit de l'éthylène est de 2,26 $lh^{-1}$ et celui de l'argon 2,74 $lh^{-1}$. La conversion en butène est de 3 %. Le catalyseur se désactive au cours du temps, et la conversion se stabilise

autour de 0,6 % après trois heures de fonctionnement.

Si après 4 heures de réaction on utilise de l'éthylène pur sous un débit de 0,735 $lh^{-1}$, la conversion en butène remonte à 10 %.

Le catalyseur se désactive à nouveau avec le temps et la conversion au bout de 3 heures se stabilise à 1 %.

Les résultats pour des réactions effectuées entre 32°C et 80°C montrent que le butène 1 est l'isomère le plus abondant en régime dynamique $\frac{\text{butène -1}}{\text{butène -2}} = 4,5$ à 32°C, l'isomérisation en butène 2 est favorisée par l'élévation de la température ou par l'augmentation du temps de contact.

Le rapport $\frac{\text{butène 2-trans}}{\text{butène 2-cis}}$ est voisin de 1 ; il diminue quand la température s'élève ou quand le temps de contact augmente.

D/ Dimérisation du propylène

La dimérisation du propylène a été étudiée sur divers catalyseurs selon l'invention, en particulier sur des catalyseurs réduits sous hydrogène photochimiquement et présentant une teneur en $Ni^{2+}$ de 1,7 et/ou de 4,9 %. La température de prétraitement sous vide était de l'ordre de 700°C.

Les conditions de dimérisation du propylène sont les suivantes :

température de réaction = 25°C
pression $C_3H_6 = 8.10^4$ Pa
temps de contact = 24 heures.

Les diverses observations ont été rassemblées dans les tableaux II et III ci-après.

TABLEAU II

Analyse des produits de la réaction (% molaire )

| $C_3H_6$ | Oléfines en $C_4$ | Oléfines en $C_5$ | Oléfines en $C_6$ |
|---|---|---|---|
| 16,4 | 1,0 | 3,8 | 78,8 |

TABLEAU III

Distribution relative des Oléfines en $C_6$ (% molaire )

| 2-3 diméthyl 2 butène | méthylpentène | n-hexène |
|---|---|---|
| 15,4 | 73,8 | 10,8 |

E/ Préparation du catalyseur contenant des ligands de blocage sélectif

Comme cela a déjà été évoqué précédemment, des phosphines, en particulier la triéthyl phosphine, la triméthyl phosphine et la triphényl phosphine, sont adsorbées en phase gazeuse sous une pression de 500 Pa à environ 2000 Pa sur le catalyseur réduit par l'hydrogène.

Dans la pratique on peut introduire sélectivement 1, 2 ou 3 molécules de phosphine dans la sphère de coordination du $Ni^+$.

Lorsque l'excès de phosphine est évacué pendant 30 minutes à environ 100°C sous vide ou sous atmosphère d'azote, on obtient la fixation d'un seul ligant phosphinique sur le $Ni^+$.

Deux complexes du nickel I sont identifiés par RPE sur le catalyseur. Chacun d'eux correspond à la présence d'une molécule de phosphine liée par cation $Ni^+$. Les caractéristiques RPE de ces deux complexes de surface sont :

espèce A : $g_{//} = 2,335$ $g_{\perp} = 2,088$

$A_{//} = 70$ G $A_{\perp} = 145$ G

espèce B : $g_{//} = 2,22$ $g_{\perp} = 2,083$

$A_{//} = 65$ G $A_{\perp} = 20$ G

Dans le cas d'une évacuation de l'excès de molécule de phosphine pendant 10 minutes à température ambiante, on observe la fixation de deux ligands phosphiniques dans la sphère de coordination du $Ni^+$.

Pour obtenir l'introduction de trois molécules de phosphine dans la sphère de coordination du $Ni^+$, il est nécessaire de conduire l'opération d'adsorption à une pression plus élevée de l'ordre de 2000 Pa. Les complexes supportés incorporant deux ou trois molécules de phosphine dans la sphère de coordination du $Ni^+$ ont été caractérisés par RPE de la façon suivante :

$Ni\left[P(Et)_3\right]_2^+$ $g_{\perp} = 2,28$ $g_{//} = 1,985$

$A_{\perp} = 40$ G $A_{//} = 50$ G (pour une phosphine)

$A_{\perp} = 70$ G $A_{//} = 90$ G (pour l'autre phosphine)

$Ni\left[P(Et)_3\right]_3^+$ $g_{//} = 2,255$ $g_{\perp} = 2,082$

$A_{//} = 40$ G $A_{\perp} = 40$ G

Un tel type de catalyseur selon l'invention comprenant une molécule de phosphine par ion $Ni^+$ permet d'obtenir une meilleure sélectivité au cours de l'oligomérisation. Ceci ressort notamment du tableau IV figurant ci-après qui établit la comparaison entre les produits réactionnels de la dimérisation de l'éthylène en utilisant respectivement un catalyseur selon l'invention exempt de phosphine et contenant un ligand triéthyl phosphine par ion $Ni^+$.

TABLEAU IV

| | % molaire | | | | |
|---|---|---|---|---|---|
| | éthylène | 1-butène | 2-butène | isobutène | hexène |
| $Ni^+/SiO_2$ | 8,6 | 2 | 43,2 | 0 | 46,2 |
| $Ni(P\ Et_3)^+/SiO_2$ | 14 | 3 | 76 | 0 | 8 |

Conditions réactionnelles : température de réaction : 25°C, temps de contact : 24 heures, Pression $C_2H_4$ : $8.10^4$ Pa, catalyseur 4,9 % en poids de $Ni^+/SiO_2$, réduction photochimique et prétraitement à 700°C.

Il ressort clairement du tableau IV ci-dessus que la présence de ligand de blocage sélectif dans la sphère de coordination du $Ni^+$ modifie de façon frappante la sélectivité du catalyseur selon l'invention. Il est également possible de tirer profit de cette propriété pour modifier la distribution relative des oléfines en $C_6$ résultant de la dimérisation du propylène.

Bien entendu, la présente invention ne saurait être limitée aux exemples particuliers décrits ci-dessus, mais il est parfaitement possible, sans pour autant sortir du cadre de la présente invention, d'en imaginer un certain nombre de variantes d'exécution.

## REVENDICATIONS

1/ Catalyseur d'oligomérisation à base de nickel sur support de silice, caractérisé en ce qu'il présente des précurseurs de sites d'activité catalytique, constitués par des complexes de nickel monovalent $Ni^+$ tricoordiné à trois atomes d'oxygène de la surface du support de silice.

2/ Catalyseur selon la revendication 1, caractérisé en ce qu'il contient d'environ 0,01 % à environ 1 % en poids sec de $Ni^+$.

3/ Catalyseur selon l'une des revendications 1 ou 2, caractérisé en ce que lesdits complexes de nickel monovalent $Ni^+$ peuvent en outre comprendre de 1 à 3 ligand(s) de blocage sélectif.

4/ Catalyseur selon la revendication 3, caractérisé en ce que lesdits complexes de nickel monovalent $Ni^+$ comprennent chacun un ligand de blocage sélectif.

5/ Catalyseur selon l'une des revendications 3 ou 4, caractérisé en ce que les ligands de blocage sélectif sont choisis parmi l'oxyde azotique et les phosphines telles que les tri-alcoyl inférieur phosphines et les tri-aryl phosphines, en particulier la tri-éthyl phosphine et la tri-phényl phosphine.

6/ Procédé de fabrication d'un catalyseur selon l'une des revendications 1 à 5, caractérisé en ce que l'on effectue les différentes étapes successives suivantes :

a) on traite la silice par une solution aqueuse d'un composé générateur de cations monovalents, à un pH supérieur à environ 10, de manière à fixer au moins une partie desdits cations monovalents sur la silice ;

12/ Procédé selon l'une des revendications 6 à 11, caractérisé en ce que l'agent réducteur utilisé au cours de l'étape de réduction d) est choisi parmi l'hydrogène, le monoxyde de carbone, l'oxyde azoteux et l'éthylène.

13/ Procédé selon la revendication 12, caractérisé en ce que l'étape de réduction d) est effectuée à une température comprise entre environ 100 et environ 300°C.

14/ Procédé selon l'une des revendications 6 à 11, caractérisé en ce que l'agent réducteur utilisé au cours de l'étape de réduction d) est choisi parmi les métaux alcalins, les dérivés organo-métalliques et les hydrures de métaux alcalins, en solution dans un milieu polaire aprotique.

15/ Procédé selon l'une des revendications 6 à 14, caractérisé en ce que l'étape de réduction d) est poursuivie jusqu'à conversion d'au moins environ 5 % du nickel divalent en nickel monovalent.

16/ Procédé selon l'une des revendications 6 à 15, caractérisé en ce que les ligands de blocage sélectif sont fixés dans la sphère de coordination du nickel par adsorption sous une pression d'environ 500 Pa à environ 2000 Pa.

17/ Procédé selon la revendication 16, caractérisé en ce que les ligands sont fixés postérieurement à l'étape de réduction d).

18/ Procédé selon la revendication 16, caractérisé en ce que la fixation des ligands est réalisée simultanément à l'étape de réduction d).

19/ Procédé d'oligomérisation, et plus particulièrement de dimérisation d'oléfines catalytique hétérogène, caractérisé en ce qu'il est mis en oeuvre en présence d'un catalyseur d'oligomérisation à base de nickel sur support de silice selon l'une des revendications 1 à 5.

b) on traite la silice ainsi obtenue par une solution aqueuse d'un sel de nickel divalent et d'un agent complexant du sel de nickel à un pH au moins égal à environ 8, pour échanger de manière compétitive lesdits cations monovalents portés par la silice avec des cations de nickel divalents ;

c) on porte la silice ainsi modifiée à une température comprise entre environ 300 et environ 900°C, et

d) on traite la silice résultante par un agent réducteur, de manière à convertir au moins une partie du nickel divalent en nickel monovalent.

7/ Procédé selon la revendication 6, caractérisé en ce que le composé générateur de cations monovalents utilisé au cours de l'étape de fixation a) est choisi parmi les hydroxydes et/ou les sels de métaux alcalins d'ammonium ou d'ammonium quaternaire.

8/ Procédé selon l'une des revendications 6 et 7, caractérisé en ce que l'agent complexant utilisé au cours de l'étape d'échange compétitif b) est choisi parmi l'ammoniac et les amines.

9/ Procédé selon la revendication 8, caractérisé en ce qu'au cours de l'étape d'échange compétitif b) le pH est compris entre environ 9 et environ 11,8.

10/ Procédé selon l'une des revendications 6 à 9, caractérisé en ce que l'étape de prétraitement thermique c) est conduite à une température comprise entre environ 500 et environ 700°C.

11/ Procédé selon l'une des revendications 6 à 10, caractérisé en ce que l'étape de prétraitement thermique c) est effectuée en présence d'un gaz renfermant de l'oxygène moléculaire.